(19) Europäisches Patentamt
European Patent Office
Office européen des brevets

(11) Publication number: **0 350 127**
**A2**

(12) **EUROPEAN PATENT APPLICATION**

(21) Application number: 89201765.8

(51) Int. Cl.4: **A61F 2/30 , A61L 27/00**

(22) Date of filing: 04.07.89

(30) Priority: 08.07.88 NL 8801730
11.05.89 NL 8901181

(43) Date of publication of application:
10.01.90 Bulletin 90/02

(84) Designated Contracting States:
AT BE CH DE ES FR GB IT LI NL SE

(71) Applicant: STAMICARBON B.V.
Mijnweg 1
NL-6167 AC Geleen(NL)

(72) Inventor: Nix, Frederik Ernst
Silhof 23
NL-6418 JV Heerlen(NL)

(54) **Plastic device for orthopedic uses.**

(57) The invention relates to an orthopedic device for replacing or connecting fragments of human or animal bone, which orthopedic device consists, at least in part, of a liquid-crystalline polymer and of which device the modulus of elasticity is at least 15 GPa in longitudinal direction and at least 10 GPa in transverse direction.

FIG. 2

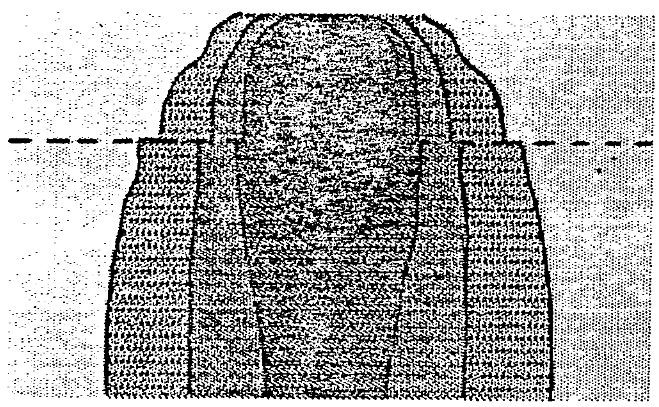



Xerox Copy Centre

## PLASTIC DEVICE FOR ORTHOPEDIC USES

The invention relates to a device for orthopedic uses, which device consists, at least in part, of plastic.

A device for orthopedic uses is understood to mean in this connection an element for replacing or connecting fragments or parts of human and/or animal bones.

An orthopedic device for man and animal is described in CRC, Critical Reviews in Biocompatibility, volume 3, Issue 4 (1987). Herein orthopedic devices are described based on metal alloys, aluminium oxide-ceramics and metal-plastic. The plastics mentioned include polyethylene and PTFE (TeflonR).

Some important properties essential for an orthopedic device are biocompatibility, resistance against wear, impact resistance, elasticity and biostability.

An example of the forces exerted on a hip is given in the Journal of Biomechanics, 1978, Vol. 11, page 75 ff. (R.D. Crowninshield et al., "A Biomechanical Investigation of the Human Hip'). From it, it may be concluded that hip prostheses must cope with a multitude of strong forces and transmit these to the bone.

From stress analyses as described in Non-Cemented Total Hip Arthroplasty, Chapter 24, page 283 ff. (Raven Press, 1988) it is known that these forces give rise to heavy loads on the cementing between prosthesis and bone. From the same sources it is known also that the prosthesis, compared with the normal situation, causes a reduction of the mechanical stresses in the cortical bone. It has been found also that both the heavy loads on the cementing and the reduced load in the cortical bone depend on the elastic properties of the material of the prosthesis.

As described in Non-Cemented Total Hip Replacement, Chapter 3, page 23 ff. (Raven Press, 1988) heavy loads on the prosthesis-bone interface may give rise to fracture and degradation of the bone.

From Biological Fixation in Total Hip Arthroplasty (Thorofare, New York: Slack Inc., 1985) it appears that reduced stresses in the cortical bone cause the bone to dissolve and the prosthesis-bone composite to weaken.

The materials applied for the prostheses now in use are responsible in part for the failure of the fixation, so that hip prostheses must often be replaced already after 10 to 15 years.

That is why there is a need of new plastic material for orthopedic devices with a high mechanical compatibility, of which material the mechanical properties are different in longitudinal and transverse directions, so that the disadvantages that occur in the use of isotropic material are avoided.

The orthopedic devices according to the invention are characterized in that the plastic is a liquid crystalline polymer having anisotropic properties.

It has been found that in the use of such a plastic in an orthopedic device the plastic shows properties virtually equivalent to the properties of bone, so that a substantial improvement is achieved of the integration of the device with the bone element.

Plastics having anisotropic properties show a high degree of orientation in the melt (thermotropic) or in a solution (lyotropic), that is the polymer chains are arranged in parallel (anisotropy). Such plastics have excellent mechanical properties in the longitudinal direction of the polymer chains. The mechanical properties perpendicular to the polymer chains differ considerably from these. Generally, the mechanical properties perpendicular to the polymer chains are lower by a factor of 10 to 7. This depends on the chemical structure of the polymer and on the existing orientation.

Details of the circumstances in which anisotropy may occur in the melt are known from J. Preston; Die Angwandte Makromolekulare Chemie 109/110 (1982) 1-19.

A bone is liable to a number of forces, for instance bending forces, compressive forces and torsional forces. Physically and chemi cally the bone structure is such as to be able to cope with these. From The Scientific Basis of Orthopaedics, J.A. Albright and R.A. Brand, Appleton-Century-Croft, New York, 1979, it is known that the bone is built up of an organic matrix in which collagen fibres are present.

The parallel arrangement results in properties different in longitudinal and transverse directions. For instance, of a cortical bone the modulus of elasticity in transverse direction is 2/3 of that in longitudinal direction.

The properties of the orthopedic device are preferably equivalent to the bone properties; that is that the orthopedic device must have properties depending on the orientation.

Plastics based on liquid-crystalline polymers showing anisotropy in the melt or in solution have properties corresponding with the properties of a bone, cortical or sponge-like.

The plastic in the orthopedic device according to the invention is preferably a thermotropic liquid-crystalline polymer.

It has been found that a thermotropic liquid-crystalline polymer shows anisotropic properties and that, with the correct modification, it has properties equivalent to those of the bone. The liquid-crystalline polymer

also has a high degree of mechanical compatibility in respect of the bone structure, which is favourable for the durability of the bone element that is in contact with the device. The result is that a long(er) residence time of an orthopedic device in a body causes fewer complications. This creates the possibility of implanting the device already at an young age of the patient without having to be replaced afterwards.

Another advantage of the orthopedic device according to the invention is the ease and flexibility of design. By flexibility of design is meant adjustment of the device both before or during the implantation by the relative softness of the plastic. It should be pointed out that thermotropic liquid-crystalline polymers are known per se.

Thermotropic liquid-crystalline polymers are generally built up from rigid aromatic units:

(a) units with an oxy and a carboxyl group,

(b) units with two oxy groups, and

(c) units with two carboxyl groups.

The choice of the unit with an oxy and a carboxyl group depends on the desired properties of the polymer. Generally, such a unit has a formula:

$$O{-}R{-}\overset{\overset{O}{\|}}{C},$$

where R contains at least an aromatic ring, for instance:

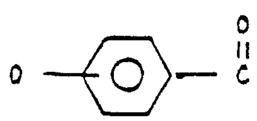

(I)

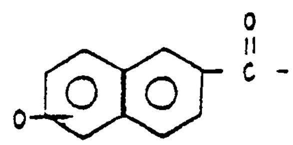

(Ia)

The liquid crystalline polymer preferably contains oxybenzoyl units with formula (I). These units come from hydroxybenzoic acid or from derivatives of these. The oxybenzoyl unit of (I) may be para or meta-substituted. Mixtures of para and meta-substituted oxybenzoyl units with formula (I) are possible also. Unit (I) is preferably para-substituted. In the aromatic ring one or more of the hydrogen atoms may be substituted by an alkyl or alkoxy group, with one to four carbon atoms, by a halogen, for instance chlorine, bromine and fluorine, and/or by a phenyl group which is substituted, if so desired. The oxybenzoyl unit is preferably derived from p-hydroxybenzoic acid.

The polymer preferably contains 20-70 moles % oxybenzoyl units calculated on the whole polymer.

Units with two oxy groups generally have a formula O-R-O, where R contains at least an aromatic ring; examples are:

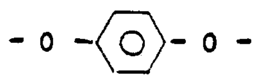

(IIa)

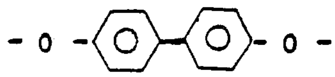

(IIb)

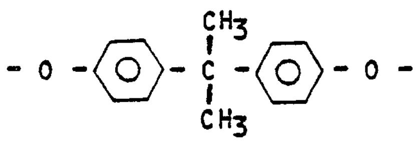

(IIc)

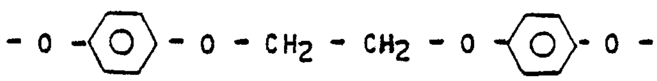

(IId)

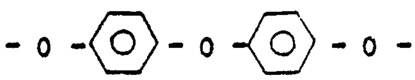

(IIe)

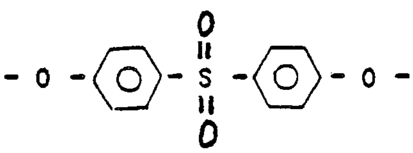

(IIf)

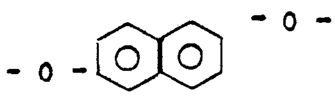

(IIg)

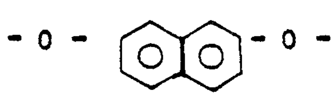

(IIh)

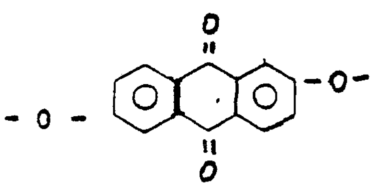

(IIi)

The liquid crystalline polymer may also contain mixtures of the above-mentioned units. The liquid crystalline polymer preferably contains dioxybenzyl (IIa) and/or biphenoxy (IIb) groups, more particularly a biphenoxy group with formula (IIb). The last-mentioned compound can be obtained from p,p′-biphenol. In the aromatic rings one or more hydrogen atoms may be substituted by an alkyl and/or alkoxy group, a halogen and/or a phenyl group, which itself may be substituted, too. The polymer preferably contains 5-40 moles % units with two oxy groups.

The aromatic units with two carboxyl groups can be obtained from aromatic dicarboxylic acid or from the corresponding esters, for instance terephthalic acid, isophthalic acid, 2,6-naphthalenedicarboxylic acid, 2,7-naphthalenedicarboxylic acid, bibenzoic acid, 4,4′-dicarboxyldiphenylsulphone, 4,4′-dicarboxyldiphenylethane, 4,4′-dicarboxyldiphenylsulphide, 4,4′-dicarboxylphenylether, 4,4′-dicarboxyldiphenylmethane, 4,4′-dicarboxyldiphenoxyethane, 2,2-bis- (4-carboxylphenyl)-propane.

The aromatic units with two carboxyl groups have preferably been obtained from terephthalic acid, isophthalic acid and/or from the derivatives thereof. In the aromatic ring one or more hydrogen atoms may be substituted by an alkyl and/or alkoxy group, a halogen and/or a phenyl group, which last-mentioned group may be substituted, too. The units with two carboxyl groups are preferably obtained from unsubstituted terephthalic acid and/or isophthalic acid. The polymer preferably contains 5-40 moles % units with two carboxyl groups.

If so desired, the polymer may also contain 0-30 moles % of an amine-containing unit, which can be

obtained from known substances such as, for instance, p-aminobenzoic acid, p-amino-phenol, p-N-methylaminophenol, p-phenylenediamine, N-methyl-p-phenylenediamine, N,N′-dimethyl-p-phenylenediamine, m-amino-phenol, 4-amino-1-naphthol, 4-amino-4′-hydroxydiphenyl, 4-amino-4′-hydroxyphenylether, 4-amino-4′-hydroxydiphenylmethane, 4-amino-4′-hydroxydiphenylethane, 4-amino-4′-hydroxydiphenylsulphone, 4-amino-4′-hydroxydiphenylsulphide, 4,4′-diaminophenylsulphide, 4,4′-diaminodiphenylsulphone, 2,5-diaminotoluene, 4,4′-ethylenediamine, 4,4′-diaminodiphenoxyethane, 4,4′-diaminodiphenylmethane, 4,4′-diaminodiphenylether.

As the use of the orthopedic device may require, such a polymer may also contain urethane, carbonate, anhydride and/or amide groups.

The preparation of the liquid-crystalline polymer according to the invention may be effected as known in the art by means of a polycondensation reaction, by reacting the monomers to be used with each other after acetylation in the desired amounts in an inert atmosphere at elevated temperature and reduced pressure. Preferably in the presence of a catalyst, for instance magnesium- and/or zinc acetate.

The properties of liquid-crystalline polymers in the orthopedic device according to the invention can be varied by chemical modification of the polymer. This can be achieved by incorporating alkyl groups with 1-3 C atoms or halogen atoms in the aromatic ring, or by introducing molecules with two aromatic rings separated by an alkyl group, an arylalkyl group or an alkoxy group. Examples of such modifications are described in US-A-4279803; US-A-4238599 and US-A-4159365.

Another possibility of adjusting the properties of the liquid-crystalline polymer is mixing the polymer with a thermoplastic polymer as described, for instance, in EP-A-30417.

The adjustment of the properties of a liquid crystalline polymer depends on the replacing or connecting fragment of the human or animal bone. The properties of the plastic in the device must preferably closely correspond with the bone properties of the area of application.

The liquid-crystalline polymer can be used in an orthopedic device as a self-reinforced plastic, as in-situ reinforced material in a mixture, as filler or in combination with other materials.

The orthopedic device according to the invention may, for instance, be a joint prosthesis, a bone plate, Kirchner or Küntscher pins, or screws. The device according to the invention is preferably a joint prosthesis, more particularly a hip prosthesis.

The modulus of elasticity of a hip prosthesis is a measure of coping with the mechanical forces which a hip prosthesis is liable to. The modulus of elasticity of the hip prosthesis is preferably at least 15 GPa in longitudinal direction and at least 10 GPa in transverse direction.

Processes for the production of an orthopedic device according to the invention are known per se. Preferably use is made of injection moulding. Liquid-crystalline polymers allow themselves to be injection-moulded quite readily, so that a prosthesis can be produced in the mould of the injection moulding machine direct.

Further, during the implantation, the orthopedic device allows itself to be readily reinforced in situ. During the implantation bore holes can be made providing a proper fit for screws or pins. The invention is further elucidated by the following description, in which reference is made to the attached drawings 1 up to and including 4. In the drawings is

fig. 1 a diagrammatic representation of a prosthetic plate applied to a bone, a force having been applied in perpendicular direction to the prosthesis,

fig. 2 a diagrammatic representation of the stress distribution in the prothesis and bone resulting from the force applied to the prosthesis, the prosthesis being made of a liquid-crystalline polymer,

fig. 3 a diagrammatic representation as in fig. 2, but now the prosthesis is made from titanium, and

fig. 4 a diagrammatic representation of the stress distribution in the medial and lateral cortex of a hip prosthesis.

Stress analyses have shown that a prosthesis under load fixed to bone (fig. 1) creates an inhomogeneous stress distribution in prosthesis and bone. If the prosthesis is made of a thermotropic liquid crystalline polymer having the same elastic properties as bone, the stress distribution will be continuous over the prosthesis-bone interface (fig. 2). The dotted line in fig. 2 is the interface between the prosthesis and the bone. The liquid crystalline polymer is obtained from units of p-hydroxybenzoic acid, terephthalic acid and biphenol (2:1:1). If, however, the prosthesis is made from conventional material, such as titanium, the stress distribution will be discontinuous (fig. 3).

Stress analyses have also shown that a hip prosthesis made from material with the same elastic properties as bone causes less stress reduction of the cortex than a prosthesis made from titanium, as indicated in Fig. 4. In this figure, the line marked with dots is the stress distribution of a prosthesis produced from anisotropic material; the non-marked line is the stress distribution of a prosthesis made from titanium.

## Claims

1. Device for orthopedic uses, which device consists, at least in part, of plastic, the device being characterized in that the plastic is a liquid-crystalline polymer having anisotropic properties.

2. Device according to claim 1, characterized in that the polymer is a thermotropic liquid-crystalline polymer.

3. Device according to any one of the claims 1-2, characterized in that the liquid-crystalline polymer contains:

a. 20-70% (wt) units derived from parahydroxybenzoic acid,

b. 5-40% (wt) units derived from biphenol and/or hydroquinone,

c. 5-40% (wt) units derived from terephthalic and/or isophthalic acid.

4. Device according to any one of the claims 1-3, characterized in that the polymer has an E-modulus of at least 15 GPa in longitudinal direction and 10 GPa in transverse direction.

5. Hip prosthesis as an orthopedic device according to any one of claims 1-4.

6. Use of liquid-crystalline polymer for the production of implantation prostheses.

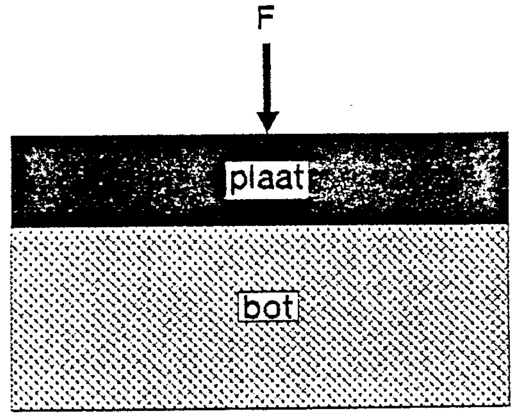


FIG. 1

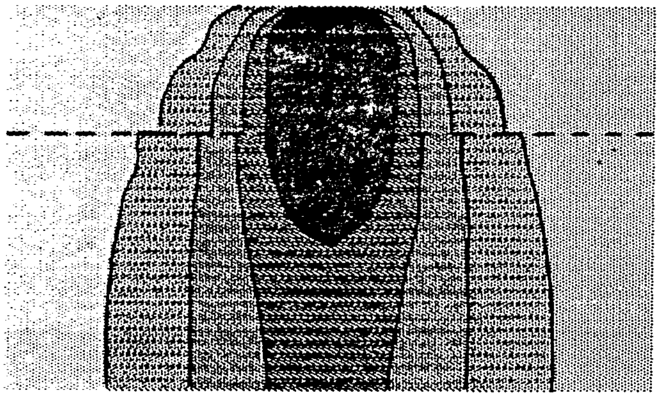

FIG. 2

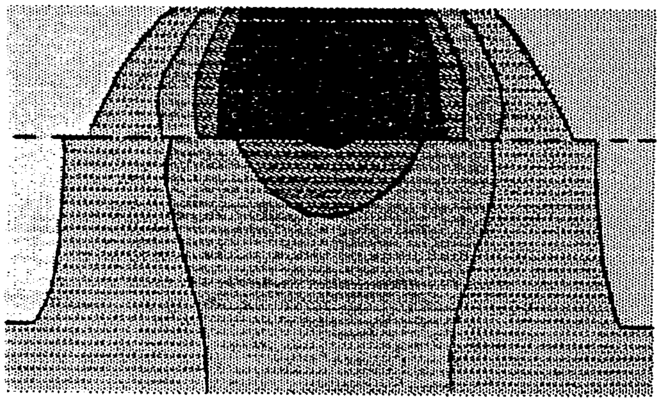

FIG. 3

BENDING STRESS CAUSED BY A BENDING MOMENT OF 1 NMM

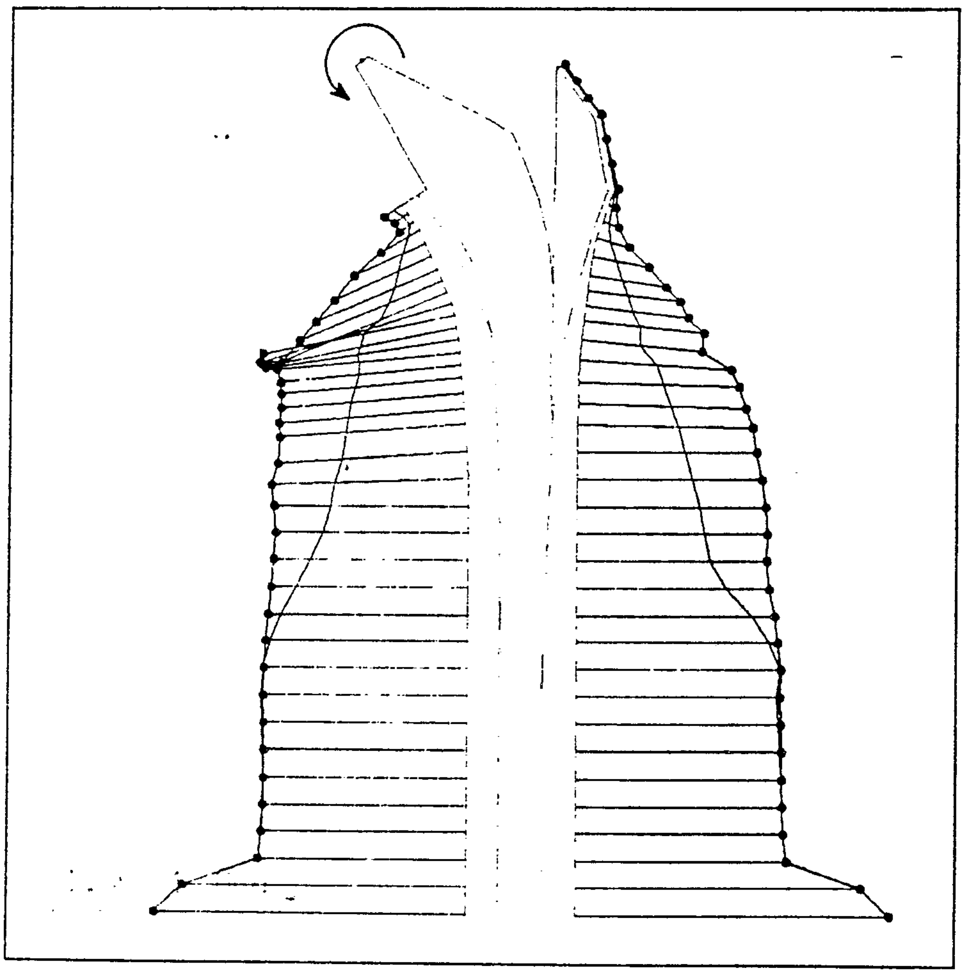

FIG. 4